# EUROPÄISCHE PATENTSCHRIFT

(11) **EP 1 741 487 B1**
(45) Veröffentlichungstag und Bekanntmachung des Hinweises auf die Patenterteilung: **09.03.2011**
(21) Anmeldenummer: 05014563.0
(22) Anmeldetag: 05.07.2005
(51) Int. Cl.: B01L 3/00, G01N 13/00, G01N 33/569, C12M 1/34, C12M 3/00

(54) **Mikrofluid-Vorrichtung und Verfahren zur Erzeugung diffusiv aufgebauter Gradienten**
Microfluidic device for generating diffusion gradients and method therefor
Dispositif microfluidique de génération des gradients de diffusion et procédé correspondant

(43) Veröffentlichungstag der Anmeldung: 10.01.2007
(60) Teilanmeldung: 10013000.4
(73) Patentinhaber: ibidi GmbH, 82152 Martinsried (DE)
(72) Erfinder: Zantl, Roman, 85598 Baldham (DE)
(74) Vertreter: Grünecker, Kinkeldey, Stockmair & Schwanhäusser Anwaltssozietät

(56) Entgegenhaltungen:
- WO-A-96/14933
- WO-A-03/078565
- WO-A-2005/079985
- US-A- 5 302 515
- US-A- 5 422 270
- US-A1- 2003 003 570
- US-B1- 6 238 874

## Beschreibung

Die Erfindung betrifft eine Mikrofluid-Vorrichtung und ein Verfahren zur Erzeugung diffusiv aufgebauter Gradienten, insbesondere im Bereich der Mikrofluidik.

Konzentrationsgradienten bestimmter Substanzen in flüssigen Medien sind für viele Anwendungen von entscheidender Bedeutung. So ist z.B. bekannt, dass für die isoelektrische Fokussierung von Proteinen ein definierter pH-Gradient benötigt wird.

Auch im Bereich der Zellbiologie sind definierte Konzentrationsverläufe für die Forschung von hoher Bedeutung. So kann z.B. die genaue Konzentration, bei der eine Substanz auf eine Zellkultur toxisch wirkt, dadurch bestimmt werden, dass in dem Nährmedium über einem homogenen Zellrasen eine kontinuierlicher Konzentrationsanstieg der zu untersuchenden Substanz aufgebaut wird.

Ein definierter und langzeitstabiler (über mehrere Stunden bis Tage) Gradient ist ebenfalls nötig, um das chemotaktische Vertialten von langsamen Zellen zu untersuchen, z.B. indem man die Migration von Zellen mit einer mittleren Geschwindigkeit von z.B. 20µm/h in oder gegen die Richtung eines Konzentrationsgradienten beobachtet. So haben z.B. einige Differenzierungen von Zellen in lebenden Organismen die Fähigkeit, sich in Richtung der Quelle (Punkt oder Raum hoher Konzentration im Gegensatz zu Senke, einem Punkt oder Raum geringerer Konzentration) von bestimmten Substanzen zu bewegen, was allgemein als "Chemotaxis" bezeichnet wird. Auf diese Weise können sich z.B. Leukozyten an Entzündungsherden anreichem und Gefäßvorläuferzellen neue Gefäße in den Regionen bilden, die z.B. mit Sauerstoff oder Nährstoffen unterversorgt sind. Die Unterbindung dieses Mechanismus gilt als aussichtsreicher Ansatz in der Bekämpfung von schnell wachsenden Tumoren. So könnte man versuchen, die Versorgung mit Nährstoffen und Sauerstoff im wuchernden Gewebe zu verhindern. Darüber hinaus spielt die Chemotaxis von Tumorzellen eine wichtige Rolle bei der Metastasierung. Somit sind insbesondere quantitative Messungen des chemotaktischen Verhaltens von Zellen des menschlichen Körpers von Interesse, die sich langsam, d.h. mit einer Geschwindigkeit von ca. 20µm pro Stunde auf die Quelle des Botenstoffes zu bewegen.

Um aussagekräftige Daten zu erhalten, wird angenommen, dass vorzugsweise Wege der Zellen von etwa dem 20-fachen des Zelldurchmessers beobachtet werden sollten. Typische Zelldurchmesser liegen zwischen 5µm und 30µm. Um etwa 600µm zurückzulegen, benötigt eine Zelle mit einer mittleren Geschwindigkeit von 20µm/h etwa 30 Stunden, was einem typischen Beobachtungszeitraum entspricht.

Der Stand der Technik bezüglich des Aufbaus eines Konzentrationsgradienten wird im Folgenden anhand des Aufbaus in Experimenten für chemotaktische Untersuchungen dargestellt.

Die von Boyden entwickelte Kammer (Boyden, S., 1962: "The chemotactic effect of mixtures of antibody and antigen on polymorphonuclear leucocytes", J Exp Med 115: 453-466) ist in verschiedenen Formen bis heute in Gebrauch. Bei diesem System trennt eine poröse Membran zwei Kammern, in denen sich unterschiedliche Konzentrationen von Chemokinen befinden. Im Membranbereich bildet sich ein stufenartiger bis sigmoider Konzentrationsgradient aus. Die Zellen werden auf eine Seite der Membran aufgebracht und bewegen sich aktiv auf die andere Seite, indem sie durch die Poren wandem. Nach einer bestimmten Zeit demontiert man die Kammer, entfemt den Filter und zählt die Zellen nach einem Färbeschritt auf der Ober- und Unterseite der Membran aus.

Schwierig bei der Auswertung ist die Unterscheidung zwischen Chemotaxis und efiöhter zufälliger Bewegung (Chemokinese). Weiterhin kann es passieren, dass migrierte Zellen sich von der Membranobertläche lösen und damit das Ergebnis verfälschen. Nur mit parallel durchgeführten Kontrollen lassen sich Aussagen darüber treffen, ob die Zellen tatsächlich auf einen Reiz reagiert haben und deswegen gewandert sind oder ob sie sich bloß zufällig auf die andere Seite der Membran bewegt haben. Mikroskopische Beobachtungen der Zellen während des Experimentes sind nicht möglich.

Eine weitere Methode ist die Verwendung von Mikrokapillaren. Bei diesem System bringt man eine Mikrokapillare mit einer mikroskopisch kleinen Öffnung in die Nähe der Zellen, die sich in einem Zellkulturgefäß befinden. Die Öffnung der Kapillare und das die Zellen umgebende Medium sind dabei fluidisch verbunden (Gerisch und Keller, 1981). Das Chemokin diffundiert bzw. strömt aus der Öffnung der Kapillare, die sich in der Nähe einer Zelle oder eines Zellverbands befindet.

Die Kapillaren sind aus Glas und müssen dementsprechend vorsichtig behandelt werden. Weiterhin ist für die Handhabung der Kapillare in der Nähe der Zellen ein Mikromanipulator nötig. Als weiterer Nachteil bei diesem System sind die hohen Kosten und die Anwenderunfreundlichkeit zu nennen. Außerdem ist die radiale Form des Gradienten um die Kapillaröffnung nur für die gleichzeitige Beobachtung einzelner oder weniger Zellen geeignet. Der Gradient kann lokal sehr steil eingestellt werden. Die Form und der Zeitverlauf des Gradienten sind komplex und nicht quantifizierbar. Auch führen kleinere Strömungen innerhalb des Zellkulturgefäßes zu massiven, nicht quantifizierbaren Konzentrationsänderungen. Solche Strömungen können z.B. durch Konvektion entstehen. Quantitative Aussagen über das Migrationsverhalten vieler Zellen sind generell nur mit mehreren parallelen Experimenten möglich, die allerdings auf Grund der komplizierten Handhabung nicht identisch durchführbar und damit nicht untereinander vergleichbar sind. Weiterhin ist für die Auswertung der Daten Bildverarbeitung nötig.

Die Zigmond-Kammer (Zigmond, S. H., 1977: "Ability of polymorphonuclear leukocytes to orient in gradients of chemotactic factors", J Cell Biol 75: 606-16; Zigmond, D. H., 1988: "Orientation chamber in chemotaxis", Methods in enzymology 162: 65-72) besteht aus zwei Kammern, die durch ein dünnes Beobachtungsvolumen zwischen den Kammern getrennt werden. Das Beobachtungsvolumen wird von oben durch ein Deckglas begrenzt, das durch geeignete mechanische Anbringung in einem definierten Abstand von der Oberfläche montiert wird. Der Wasserstand in den beiden Kammern muss möglichst genau der Höhe des Deckglases entsprechen. In beiden Kammern befindet sich identisches Zellnährmedium, wobei zusätzlich in einer der Kammern eine definierte Menge des Chemokins zugemischt wird. In dem Sichtfenster bildet sich im Idealfall durch Diffusion ein linearer chemischer Gradient aus. Die Verwendung des Deckglases ermöglicht es, die chemotaktische Bewegung der Zellen mikroskopisch zu verfolgen.

Eine Einschränkung für derartig durchgeführte Untersuchungen besteht darin, dass Zellen auf Gradienten nur dann reagieren können, wenn der Konzentrationsabfall über eine Zelllänge etwa 1% der durchschnittlichen Konzentration am Ort der Zelle beträgt. Das bedeutet, dass der Gradient ausreichend steil sein muss. Durch Diffusion und unerwünschten Fluss ist der Gradient in realen Zigmond-Kammem etwa 30 bis 60 Minuten lang steil genug für Chemotaxisstudien. Danach ist er zu stark abgeflacht oder durch Strömungen gestört. Darüber hinaus erfordert die Anwendung der Zigmond-Kammer ein hohes Geschick bei der Durchführung entsprechender Versuche, da durch den mehrteiligen Aufbau und die offenen Kammern sehr schnell Flüssigkeitsströmungen den Gradienten zerstören können.

Die Dunnkammer (Zicha, D., G. A. Dünn und A. F. Brown, 1991: "A new directviewing chemotaxis chamber", J Cell Sci 99 (Pt 4): 769-75.) ist eine Weiterentwicklung der Zigmond-Kammer. Die beiden Bereiche, die das Chemokin enthalten bzw. nicht enthalten sind radial zueinander angeordnet und durch eine geschlossene, ringförmige Barriere voneinander getrennt. Nach Auflegen des Deckglases befindet sich der zur Untersuchung dienende Spalt zwischen der ringförmigen Barriere und dem Deckglas, so dass untersucht werden kann, ob die Zellen gezielt z.B. zum Zentrum der Anordnung laufen, wenn im Mittelbereich das Chemokin zugemischt wurde.

Die Dunnkammer erlaubt die chemotaktische Untersuchung über längere Zeiträume als die Zigmond-Kammer. Nachteilig ist hierbei die Geometrie, da die zu erwartende Richtung der chemotaktischen Bewegung vom Ort im Spalt abhängt. Dies verkompliziert die Auswertung der Daten. Außerdem ist der chemische Gradient sehr instabil gegenüber mechanischen Einflüssen, wie z.B. einem Schräghalten der Kammer, was u.a. zur Störung des diffusiven Gradienten durch den zusätzlich auftretenden Fluss führt.

Auch die internationale Patentanmeldung WO 03/078565 beschäftigt sich mit chemotaktischen Untersuchungen und verwendet ein Gehäuse aus einer Basis-Platte auf die eine obere Komponente derart montiert wurde, dass sie zwei Kammern definieren, die durch einen Kanal verbunden sind.

Die Druckschrift US 5 302 515 offenbart weiter eine Apparatur zur Messung von Konzentrationsgradienten und verwendet zwei Gehäuse-Teile, die derart miteinander verbunden sind, dass sich zwischen ihnen ein dünner Durchgang befindet. Die Dimensionen des Durchgangs sind dabei derart gewählt, dass die Zellen sich deformieren müssen, um durch diesen Durchgang zu migrieren.

Die Druckschrift US 2003/0003570 beschäftigt sich mit der Aufgabe, den Transfer einer Mikroprobe von einer Kammer in eine nächste Kammer kontrolliert einzustellen. Die zugehörige Apparatur umfasst Kammern, die miteinander durch einen Kanal verbunden sind, der einen Widerstand für einen Fluidstrom darstellt, und Röhren, die dazu dienen, Druck oder Unterdruck in dem System zu erzeugen.

US Patent Nr. US 6 238 874 beschreibt eine Apparatur zur Untersuchung von Zellbewegungen aufgrund eines chemotaktischen Konzentrationsgradienten. Die Apparatur umfasst eine Kammer zur Hineingabe der Zellen und eine Kammer zur Hineingabe eines chemotaktischen Mittels, so dass sich zwischen diesen Kammern ein Konzentrationsgradient des chemotaktischen Mittels einstellt. Die Migration der Zellen kann somit in Abhängigkeit des Konzentrationsgradienten untersucht werden.

Die internationale Anmeldung WO 96/14933 offenbart auch ein Gerät zur Analyse von Zellbewegungen und umfasst Kammern, Zugänge und einen Kanal zwischen den Kammern zur Beobachtung der Zellbewegungen.

Generell hängt das chemotaktische Vefialten des jeweiligen Zelltyps nicht nur von der Substanz, sondern auch von deren Konzentration und der Steilheit des Konzentrationsgefälles ab. Als Richtwert kann für das gerade noch von der Zelle erkennbare Konzentrationsgefälle - wie oben bereits erwähnt - etwa 1% Gefälle pro Zelllänge am Ort der Zelle gelten.

Als Beispiele für Zellen dienen hier insbesondere chemotakisch aktive Zellen wie Nabelschnurendothelzellen (HUVEC), die u.a. auf den Stoff VEGF (vascular endothelial growth factor) reagieren, und die Tumorzelllinie HT1080, die chemotaktisch auf FCS (fötales Kälberserum) reagiert.

Auf Grund der oben beschriebenen Nachteile im Stand der Technik ist es Aufgabe der Erfindung, eine Mikrofluid-Vorrichtung und ein Verfahren bereitzustellen zur Erzeugung diffusiv aufgebauter Gradienten, wobei die Mikrofluid-Vorrichtung und das Verfahren die Störungen des Gradienten durch Druckschwankungen und/oder Fluss in der Mikrofluid-Vorrichtung verhindern.

Diese Aufgabe wird gelöst durch eine Mikrofluid-Vorrichtung nach Anspruch 1 und durch ein Verfahren nach Anspruch 15.

Die erfindungsgemäße Mikrofluid-Vorrichtung zur Erzeugung diffusiv aufgebauter Gradienten umfasst eine Bodenplatte und eine Deckplatte, wobei die Deckplatte Aussparungen aufweist und mit der Bodenplatte flüssigkeitsdicht verbunden ist, so dass die Aussparungen mindestens zwei Reservoire und eine Beobachtungskammer, die die Reservoire verbindet, bilden, ein Reservoir, insbesondere durch einen Zu-/Ablauf durch die Deckplatte, befüllbar ist und die Querschnittsfläche der Beobachtungskammer an der Mündung der Beobachtungskammer in eines der Reservoire mindestens 5mal, insbesondere mindestens 20mal kleiner als die maximale Querschnittsfläche des Reservoirs parallel zu dieser Querschnittsfläche der Beobachtungskammer ist.

Vorzugsweise kann die Querschnittsfläche der Beobachtungskammer an der Mündung der Beobachtungskammer in eines der Reservoire mindestens 10mal, insbesondere mindestens 30mal kleiner als die maximale Querschnittsfläche des Reservoirs parallel zu dieser Querschnittsfläche der Beobachtungskammer sein.

Ein diffusiv sich aufbauender Gradient oder ein diffusiv aufgebauter Gradient ist ein Konzentrationsgradient, der sich auf Grund von unterschiedlichen Konzentrationen in zwei Räumen (z.B. zwei Reservoiren) in einem dritten Raum (z. B. einer Beobachtungskammer) zwischen den zwei Räumen aufbaut oder aufgebaut hat und der sich durch die Diffusion zwischen den zwei Räumen (die als Quelle und Senke dienen) einstellt. Für einen sich diffusiv aufbauenden Gradienten sollte vorzugsweise zusätzlich zur Diffusion kein weiterer Fluss zwischen den Räumen stattfinden (z.B. können Druckschwankungen einen zusätzlichen Fluss verursachen und die diffusive Gradientenbildung zerstören).

Falls die Querschnittsfläche eines Reservoirs nicht konstant ist, ist die Mündung die Stelle, an der die Querschnittsfläche des Reservoirs stetig oder sprunghaft einen kleinsten Wert, nämlich den Wert für die Querschnittsfläche der Beobachtungskammer an der Mündung erreicht. Falls beispielsweise ein Reservoir eine Zylinderform aufweist und die Beobachtungskammer in die gekrümmte Seitenwand des Reservoirs mündet, so wird die (plane) Querschnittsfläche der Beobachtungskammer durch die Schnittlinie der Beobachtungskammer mit dem Zylinder begrenzt. Entsprechend wird die Querschnittsfläche der Beobachtungskammer auch bei anderen gekrümmten Reservoiroberflächen bestimmt.

Im Allgemeinen ist ein Reservoir der erfindungsgemäßen Mikrofluid-Vorrichtung befüllbar, wenn man in das Reservoir eine Flüssigkeit und/oder Zellen füllen kann.

Die Bodenplatte und die Deckplatte sind flüssigkeitsdicht verbunden, was bedeutet, dass an ihren Verbindungsstellen keine Flüssigkeit ein oder austreten kann.

Die erfindungsgemäße Mikrofluid-Vorrichtung ist vorteilhaft gegenüber dem Stand der Technik, weil die flüssigkeitsdichte Verbindung zwischen Boden- und Deckplatte und die unterschiedlichen Größen der Querschnittsflächen des Reservoirs verglichen mit der Beobachtungskammer eine langzeitstabile Diffusion von der Quelle zur Senke ermöglichen.

So kann z.B. ein Reservoir eine definierte Konzentration einer chemischen Substanz enthalten, so dass dieses Reservoir als Quelle für diese Substanz dient. Das zweite Reservoir kann dann eine deutlich geringere Konzentration der chemischen Substanz enthalten und dient als Senke dieser Substanz. Die Reservoire liegen an gegenüberliegenden Seiten der Beobachtungskammer. Auf Grund des viel größeren Querschnitts der Reservoire verglichen mit dem Querschnitt der Beobachtungskammer, ist es nun möglich, dass sich zwischen Quelle und Senke ein lineares, langzeitstabiles Konzentrationsgefälle durch Diffusion der chemischen Substanz aufbauen kann.

Ein weiterer Vorteil gegenüber der Zigmond-Kammer ist, dass - obwohl die Bodenplatte mit der Deckplatte verbunden ist - ein Reservoir der Mikrofluid-Vorrichtung weiter befüllbar bleibt und somit eine einfachere und flexiblere Durchführung von Experimenten ermöglicht wird.

Vorzugsweise liegen der Boden der Beobachtungskammer und der Boden mindestens eines Reservoirs in einer Ebene.

Die Querschnittsflächen der Beobachtungskammer sind an mindestens zwei Mündungen in jeweils ein Reservoir mindestens 5mal kleiner, insbesondere mindestens 10mal kleiner, als die maximale Querschnittsfläche des Reservoirs parallel zu dieser Querschnittsfläche der Beobachtungskammer. Vorzugsweise können die Querschnittsflächen der Beobachtungskammer an mindestens zwei Mündungen in jeweils ein Reservoir mindestens 20mal kleiner, insbesondere mindestens 30mal kleiner, sein als die maximale Querschnittsfläche des Reservoirs parallel zu dieser Querschnittsfläche der Beobachtungskammer.

In einer bevorzugten Weiterbildung kann die Bodenplatte oder die Unterseite der Bodenplatte der Mikrofluid-Vorrichtung plan sein, und insbesondere kann die Bodenplatte den Boden der Reservoire, der Beobachtungskammer und eventueller Kanäle der Mikrofluid-Vorrichtung darstellen. Eine Bodenplatte kann vorzugsweise eine Folie sein, und insbesondere kann die Bodenplatte zwischen 0,2µm und 2mm dick sein. Vorzugsweise hat sie die Grundfläche eines herkömmlichen Objektträgers, z.B. 25,5mm x 75,5mm, und die Grundflächen der Bodenplatte und der Deckplatte können insbesondere identisch groß sein.

Die Deckplatte kann vorzugsweise plan sein, insbesondere im Bereich eines Zu-/Ablaufs. Außerdem kann die Deckplatte etwa zwischen 0,5mm und 3mm dick sein, vorzugsweise 1,5mm. Insbesondere können die Boden- und Deckplatte flüssigkeits- und/oder luftdicht verbunden sein. Das Volumen eines Reservoirs kann in dem Bereich von 5µl bis 1000µl, vorzugsweise zwischen 100µl und 500µl, insbesondere bei 300µl liegen.

Eine Beobachtungskammer kann eine polygonale Grundfläche als Boden und als Decke aufweisen, und insbesondere können der Boden und/oder die Decke plan sein. Der Boden wird durch die Bodenplatte der Mikrofluid-Vorrichtung, und die Decke wird von der Deckplatte der Mikrofluid-Vorrichtung gebildet. Vorzugsweise können mindestens zwei Seiten einer Beobachtungskammer jeweils mit einem Reservoir verbunden sein. Es ist möglich, dass die gesamte Fläche einer Seite der Beobachtungskammer als Öffnung zum Reservoir dient, also diese Seite der Beobachtungskammer komplett offen ist. Die Höhe der Beobachtungskammer (Abstand zwischen Boden und Decke) kann vorzugsweise zwischen 10µm bis 500µm, insbesondere 75µm betragen, die Breite vorzugsweise etwa 30µm bis 1cm, insbesondere 1 mm, betragen und die Länge (Abstand zwischen den Mündungsebenen der Reservoire) etwa 100µm bis 5mm, insbesondere etwa 500µm bis 3mm oder etwa 1 mm bis 2mm, betragen.

In einer bevorzugten Weiterbildung kann die Beobachtungskammer ein schmaler Kanal, Spalt oder eine Kapillare mit vorzugsweise geringer Höhe sein. Dadurch ist es möglich, eine vorteilhafte, große Beobachtungsfläche bei möglichst geringem Stofftransport zu erhalten. Dies führt dann zu quasi zweidimensionaler Diffusion und damit zu vereinfachten Beobachtungsmöglichkeiten. Wird ein kleiner oder schmaler Kanal als Beobachtungskammer genutzt, kann der Stofftransport durch diesen Kanal verlaufen und der Abbau des Gradienten kann sehr langsam geschehen. Die Höhe der Reservoire ist typischerweise deutlich größer als die der Beobachtungskammer, so dass auf der Seite, die als Konzentrationssenke dient, die Konzentration der diffusiv transportierten Stoffe durch die 3-dimensionale Diffusion schnell und stark verdünnt werden kann.

Vorzugsweise kann das Volumen mindestens eines Reservoirs mehr als 50mal größer, insbesondere mehr als 100mal größer, sein. Vorzugsweise kann das Volumen mindestens eines Reservoirs mindestens 200mal, insbesondere mindestens 500mal, und vorzugsweise mindestens 1000mal größer als das Volumen der Beobachtungskammer sein.

In einer vorteilhaften Weiterbildung kann die Querschnittsfläche der Beobachtungskammer zwischen 0,05mm² und 0,4mm², vorzugsweise zwischen 0,075mm² und 0,1mm², liegen und/oder die Querschnittsfläche eines Reservoirs zwischen 0,25mm² und 8mm², vorzugsweise zwischen 0,37mm² und 2mm² liegen.
Die Querschnittsfläche eines Reservoirs kann vorzugsweise zwischen 0,25mm² und 2mm², insbesondere zwischen 0,37mm² und 0,5mm² betragen, falls die Querschnittsfläche der Beobachtungskammer an der Mündung der Beobachtungskammer in das Reservoir mindestens 5mal kleiner ist als die Querschnittsfläche des Reservoirs parallel zu dieser Querschnittsfläche der Beobachtungskammer. Ist die obige Querschnittsfläche der Beobachtungskammer mindestens 20mal kleiner als die oben beschriebene Querschnittsfläche des Reservoirs, kann die Querschnittsfläche des Reservoirs vorzugsweise zwischen 1mm² und 8mm², insbesondere zwischen 1,5mm² und 2mm², betragen.

Ist die obige Querschnittsfläche der Beobachtungskammer mindestens 10mal kleiner als die oben beschriebene Querschnittsfläche des Reservoirs, kann die Querschnittsfläche des Reservoirs vorzugsweise zwischen 0,5mm² und 4mm², insbesondere zwischen 0,75mm² und 1 mm², betragen. Ist die obige Querschnittsfläche der Beobachtungskammer mindestens 30mal kleiner als die oben beschriebene Querschnittsfläche des Reservoirs, kann die Querschnittsfläche des Reservoirs vorzugsweise zwischen 1,5mm² und 12mm², insbesondere zwischen 2,25mm² und 3mm², betragen.

In einer vorteilhaften Weiterbildung können in ein Reservoir weiterhin mindestens zwei, insbesondere drei, Kanäle führen. Vorzugsweise kann das Reservoir quaderförmig sein, und die Kanäle können insbesondere parallel zueinander liegen und in die gleiche Reservoirseite führen. Vorzugsweise kann diese Reservoirseite gegenüber der Reservoirseite liegen, die an die Beobachtungskammer grenzt. Insbesondere können die Kanäle Zu-/Abläufe aufweisen.

Die obige Weiterbildung ist vorteilhaft, da durch die offenen Zu-/Abläufe der Kanäle ein Fluss in der Kanalstruktur gesteuert werden kann, ohne einen Fluss in der Beobachtungskammer zu erzeugen. Dadurch können Konzentrationen oder Konzentrationsschwankungen in der Kanalstruktur kontrolliert und/oder vermieden werden.

Zusätzlich kann jedes Reservoir mindestens einen verschließbaren und/oder befüllbaren Zu-/Ablauf aufweisen, insbesondere kann der Zu-/Ablauf flüssigkeits- und/oder luftdicht verschließbar sein und führt vorzugsweise durch die Deckplatte in ein Reservoir. Durch einen Zu-/Ablauf kann das Reservoir befüllbar sein, insbesondere mit einer Flüssigkeit, oder eine Flüssigkeit dem Reservoir entweichen oder entnommen werden. Vorzugsweise kann ein Zu-/Ablauf die Form eines Durchgangslochs durch die Deckplatte in ein Reservoir haben. Vorzugsweise können die Zu-/Abläufe eines Reservoirs luftdicht verschließbar sein. Innerhalb kleiner Volumina können die wässrigen Flüssigkeiten praktisch inkompressibel sein, so dass auftretende Druckschwankungen so gut wie keine Flüssigkeitsbewegungen hervorrufen können. Generell kann ein Zu-/Ablauf einen Kanal umfassen, der durch eine Aussparung in der Deckplatte zusammen mit der Bodenplatte gebildet ist.

Ein Zu-/Ablauf kann eine minimale Querschnittsfläche zwischen 0,03mm² und 13mm², insbesondere zwischen 0,2mm² und 3,2mm², aufweisen. Die Querschnittsfläche eines Zu-/Ablaufs kann insbesondere variieren, z.B. bei einem konischen geformten Zu-/Ablauf ist die minimale Querschnittsfläche die kleinste Querschnittsfläche, die der konische Zu-/Ablauf aufweist. Die Querschnittsfläche eines Zu-/Ablaufs kann insbesondere konstant sein, z.B. können die Abmessungen eines Zu-/Ablauf mit konstanter, z.B. rechteckiger, Querschnittsfläche 300µm x 100µm bis 2mm x 0,5mm betragen. Ein Zu-/Ablauf mit runder Querschnittsfläche kann z.B. Durchmesser zwischen 0,3mm bis 4mm, insbesondere zwischen 0,5mm und 2mm aufweisen.

Weiterhin kann ein Zu-/Ablauf, insbesondere zusätzlich zu einem Kanal, Öffnungen, z.B. konische Töpchen (insbesondere mit einer Höhe von bis zu 7mm und einem Durchmesser von bis zu 2mm im mittleren Querschnitt), für die Einführung von z.B. Pipettenspitzen oder für das Anbringen von Schläuchen oder Pumpen aufweisen. Auch können die Zu-/Abläufe so ausgebildet sein, dass ein kleiner Tropfen, der auf den Zulauf aufgebracht wird, in das Reservoir gezogen wird. Dadurch, dass der mindestens eine Zu-/Ablauf verschließbar ist, wird jeglicher Fluss durch die Beobachtungskammer und somit die Zerstörung des Gradienten verhindert.

Der oben beschriebenen Zu-/Ablauf mit seinen kleinen Querschnittsflächen stellt einen weiteren Vorteil gegenüber bestehender Zigmond- und Dunnkammem dar. Im einfachsten Fall können die Zu-/Abläufe durch Vakuumfett oder einen Klebefilm (z.B. von Tesa) flüssigkeits- und/oder luftdicht verschlossen werden. Auch können diese mit passenden Deckeln oder Steckern verschlossen werden. Durch das Verschließen mit Deckeln kann es allerdings zu Druckschwankungen und somit zu einem Fluss innerhalb der Mikrofluid-Vorrichtung kommen. Um dies zu verhindern, kann z.B. ein Deckel aus einem Stück gefertigt sein, der alle Zu-/Abläufe und sonstige Öffnungen, wie z.B. eventuelle Durchganglöcher, gleichzeitig verschließen kann.

Weiterhin kann ein Durchgangsloch durch die Deckplatte und/oder Bodenplatte in die Beobachtungskammer vorgesehen sein. Dabei kann das Durchgangsloch auch in Form einer Erhöhung (z.B. ein konischer Aufsatz, der mit der Deckplatte irreversibel verbunden ist) oder die komplette Deckplatte im Bereich der Beobachtungskammer als Durchgangsloch ausgebildet sein. Durch ein solches Durchgangsloch kann insbesondere ein Gel (zur Reduzierung der Diffusionsgeschwindigkeit und somit insbesondere zur Stabilisierung des Gradienten) oder können insbesondere Sphäroide eingefüllt werden. Sphäroide sind meist abgeplattete, kugelförmige Zellhaufen mit einem Durchmesser von 100µm bis 1mm, die durch entsprechende Präparation aus einer Vielzahl von Zelltypen geformt werden können. Das Einfüllen der Sphäroide im Gegensatz zum Befüllen mit vereinzelten Zellen kann den Vorteil haben, dass die Zellen zu Beginn des Experiments an einem Punkt konzentriert sind, was zu einer einfacheren Symmetrie des Systems führt. Wandern die Zellen von dem Symmetriepunkt bevorzugt in eine Richtung, ist das sofort ohne weitere Auswertung zu erkennen.

In einer bevorzugten Ausführungsform können die Bodenplatte und die Deckplatte irreversibel miteinander verbunden sein, insbesondere können die Bodenplatte und die Deckplatte verklebt, ultraschallgebondet, über Hitze oder ein Lösungsmittel gebondet (siehe z.B. DE 101 30 428 A1) sein.

Insbesondere kann die Bodenplatte mindestens in dem Bereich der Beobachtungskammer aus einem optisch hochwertigen Material bestehen. Optisch hochwertige Materialien weisen eine geringe Doppelbrechung und eine geringe Fluoreszenz auf, was insbesondere bei Durchführung von Fluoreszenzmikroskopie von Vorteil ist. In einer weiteren Ausführungsform können die gesamte Bodenplatte und/oder die Deckplatte aus einem optisch hochwertigen Material bestehen. Der Brechungsindex der Materialien liegt dabei für optische Analysen bevorzugt zwischen 1,2 und 1,6.

In einer bestimmten Ausführungsform können sich die Reservoire an gegenüberliegenden Seiten der Beobachtungskammer befinden. Dies kann eine gleichmäßige Diffusion von dem als Quelle dienenden Reservoir zu dem als Senke dienenden Reservoir begünstigen.

Weiterhin umfasst die Mikrofluid-Vorrichtung zusätzlich mindestens einen Kanal mit mindestens einem Zu-/Ablauf, wobei der Kanal in die Beobachtungskammer führt und insbesondere sich der Boden des Kanals in der gleichen Ebene befinden kann wie der Boden der Beobachtungskammer. Der mindestens eine Zu-/Ablauf kann die gleichen Strukturen und Dimensionen aufweisen wie ein Zu-/Ablauf eines Reservoirs. Der Kanal kann z.B. 0,5mm breit sein und insbesondere die gleiche Höhe wie die Beobachtungskammer besitzen. Über diesen Kanal kann die Beobachtungskammer z.B. mit Zellen befüllt werden. Zusätzlich kann in die gegenüberliegende Seite ebenfalls ein Kanal mit z.B. gleichen Abmessungen münden. Auf diese Weise kann ein Kreuzungskanal entstehen, in dem Moleküle oder Zellen auch nach Etablierung des Gradienten in die Beobachtungskammer eingespült werden können. Unter Umständen können erst nach einer entsprechenden Wartezeit, nachdem sich wieder der quasistatische Zustand in der Beobachtungskammer eingestellt hat, aussagekräftige Untersuchungen durchgeführt werden.

Der Kanal führt über eine Seite der Beobachtungskammer in die Beobachtungskammer, wobei die Beobachtungskammer an dieser Seite nicht an ein Reservoir anschließt.

So umfasst die Mikrofluid-Vorrichtung zwei Reservoire und eine Beobachtungskammer zwischen den Reservoiren , wobei ein Kanal z.B. senkrecht zu beiden Reservoiren in die Beobachtungskammer führt. Ein eventueller zweiter Kanal kann vorzugsweise in die Beobachtungskammer an der gegenüberliegenden Seite der Mündung des ersten Kanals münden und kann insbesondere ebenfalls senkrecht zu den beiden Reservoiren vorgesehen sein, so dass beide Kanäle einen Kreuzungskanal bilden können.

Generell ist bei der Dimensionierung von Kanälen zu berücksichtigen, dass bei bestimmten Abmessungen Kapillareffekte auftreten können, so dass eine Flüssigkeit in dem Kanal eingeklemmt werden kann.

In einer bestimmten Ausführungsform kann die Höhe und/oder Breite der Beobachtungskammer und/oder der Reservoire der Mikrofluid-Vorrichtung vorzugsweise konstant sein. Insbesondere kann die Beobachtungskammer eine Höhe zwischen 0,05mm und 0,15mm und eine Breite zwischen 0,15mm und 5mm haben, und insbesondere mindestens ein Reservoir eine Höhe zwischen 0,5mm und 1 mm und eine Breite zwischen 4mm und 9mm aufweisen.

Die Beobachtungskammer und/oder die Reservoire können in einer bestimmten Ausführungsform die Form eines geraden Prismas mit einer geraden Seitenanzahl besitzen. Ein "gerades Prisma mit gerader Seitenzahl" bedeutet, dass sich die Seitenwände des Prismas senkrecht zur geradzahlig polygonalen Grund- und Deckfläche des Prismas befinden, also die Deckfläche parallel zur Grundfläche liegt und die Anzahl der rechteckigen Seitenwände gerade ist. Vorzugsweise können die Beobachtungskammer und/oder die Reservoire quaderförmig ausgebildet sein.

Insbesondere kann die Mikrofluid-Vorrichtung mindestens zwei weitere Reservoire umfassen, wobei die Reservoire an den Seiten der Beobachtungskammer so angeordnet sind, dass sich jeweils zwei Reservoire gegenüberliegen. Um die Stärke des Einflusses verschiedener Substanzen auf das chemotaktische Verfialten miteinander direkt vergleichen zu können, können auf diese Weise Reservoire mit jeweils verschiedenen Substanzen gefüllt werden, die in der Beobachtungskammer Gradienten in verschiedenen Richtungen erzeugen können.

Vorzugsweise kann die Mikrofluid-Vorrichtung wenigstens eine Zelladhäsionsfläche in der Beobachtungskammer aufweisen. Die Zelladhäsionsfläche kann in Form einer Obertlächenstrukturierung, beispielsweise durch Plasmabehandlung oder UV-Belichtung unter Zuhilfenahme einer Maskentechnik , und/oder einer Oberflächenbehandlung, beispielsweise durch Beschichtung mit Adhäsionsproteinen oder Peptiden mittels Arrayplottem, realisiert werden. Die eine oder mehrere Zelladhäsionsflächen können insbesondere insgesamt kleiner als der Boden der Beobachtungskammer sein. Zelladhäsionsflächen können die Auswertung des chemotaktischen Assays erleichtern.

Ein Beispiel für eine entsprechende Ausführungsform besteht aus einer rechteckigen Beobachtungskammerbodenfläche, insbesondere in der Größe von 1 mm x 2mm, wobei die längeren Kanten der Fläche jeweils mit einem Reservoir in der Mündungsebene verbunden sind. Parallel zu den kürzeren Kanten, also in Richtung der zu erwartenden chemotaktischen Migration, sind langgestreckte rechteckige Bereiche, insbesondere von je 100µm x 500µm, für Zelladhäsion behandelt. Die übrige Fläche des Beobachtungsbereich ist nicht weiter oberflächenbehandelt und erlaubt demnach keine Zelladhäsion. Spült man nun Zellen in die Beobachtungsfläche, lagem sich diese statistisch verteilt an den langgestreckten rechteckigen Adhäsionsbereichen an. Nach Durchführung des Chemotaxis-Experiments zeigt sich die Migration durch eine signifikante Erhöhung der Zelldichte in Richtung des Reservoirs, welches die Quelle darstellt, im Gegensatz zu einer deutlich geringeren Zelldichte in Richtung des Reservoirs, welches die Senke darstellt.

Bei einer anderen, möglichen Oberflächenstrukturierung und/oder -behandlung kann die Adhäsionsfläche etwa so breit sein wie eine adhärierte Zelle. Je nach Zelltyp beträgt der Zelldurchmesser zwischen 5µm und 20µm. Diese Struktur kann insbesondere vorteilhaft sein, wenn die Chemotaxis von Zellen untersucht werden soll, die stark auf Zellkontakte untereinander reagieren. So zeigen z. B. menschliche Endothelzellen eine stärker gerichtete Reaktion auf gleiche Zellen als auf chemische Gradienten, was die Auswertung der Experimente erschwert. Vorzugsweise können die Zellen sich wie auf einer Perlenschnur aufgereiht in einer Reihe befinden und sich nur auf die Quelle zu oder davon weg bewegen. Diese Ausführung kann dann der generellen Beurteilung dienen, ob die Zellen Chemotaxis zeigen.

Weiterhin kann die Mikrofluid-Vorrichtung wenigstens eine rechteckige, rautenförmige, dreieckige oder runde Zelladhäsionsfläche aufweisen. Dabei können die rautenförmigen und dreieckigen Adhäsionsflächen mit einer Ecke auf das Reservoir weisen, welches die Quelle darstellt. Verdichten sich die Zellen in dieser Ecke, ist Chemotaxis mit bildverarbeitender Auswertung einfach nachzuweisen.

Vorzugsweise weist die Mikrofluid-Vorrichtung wenigstens eine Markierung in der Beobachtungskammer auf zur Bestimmung von Ort und/oder Geschwindigkeit von migrierenden Zellen. Die Markierung kann eingepresst, aufgedruckt, aufgemalt, eingelasert etc. sein und kann insbesondere zur Spezifizierung einer Diffusion in der Beobachtungskammer dienen. Die Markierungen können insbesondere 1µm bis 100µm groß sein und ihr Abstand zueinander kann zwischen 10µm bis 1 mm betragen.

In einer weiteren Ausführungsform kann die Beobachtungskammer in der Mündungsebene zu den Reservoiren Strukturen oder Materialen wie z.B. Collagen oder Matrigel aufweisen. Diese können die Diffusionsgeschwindigkeit reduzieren und somit insbesondere die Gradienten länger stabil halten. Auch können die Zellen anstatt in flüssigem Medium in eine Gelmatrix wie z.B. Matrigel gefüllt werden, um die Verhältnisse im menschlichen und tierischen Körper noch besser zu simulieren.

Die Erfindung stellt weiterhin ein Analysesystem bereit, das die erfindungsgemäße Mikrofluid-Vorrichtung und eine Auslesevorrichtung, insbesondere Mikroskope oder Elektrodenarrays, umfasst.

In einer weiteren Ausführungsform können zur Unterstützung des Gradienten Elektroden in der oder an die Beobachtungskammer angebracht sein. Viele biologisch relevante Moleküle tragen eine Ladung, was dazu ausgenutzt werden kann, Konzentrationsgradienten unter Nutzung von Elektroden zu erzeugen oder die Gradienten weiter zu stabilisieren.

Generell kann die erfindungsgemäße Mikrofluid-Vorrichtung aus einem Kunststoff, wie z.B. PC (Polycarbonat), PS (Polystyrol), PE (Polyethylen), PVC (Polyvinylchlorid), COC (Cyclo-olefin Copolymer), COP (Cyclo-olefin Polymer), PMMA (Polymethylmethacrylat) etc. bestehen. Je nach Anwendung können gasdurchlässige oder gasundurchlässige Kunststoffe verwendet werden.

Eine besonders bevorzugte Ausführungsform ist eine Anordnung zur Erzeugung diffusiv aufgebauter Gradienten mit wenigstens zwei der oben beschriebenen Mikrofluid-Vorrichtungen, wobei die wenigstens zwei Bodenplatten als eine einzige Bodenplatte ausgebildet sind und die wenigstens zwei Deckplatten als eine einzige Deckplatte ausgebildet sind. Die Anordnung kann z.B. das Format eines Objektträgers oder einer Multiwellplatte haben, d.h. sie kann insbesondere 86mm x 128mm groß sein. Dabei können die Beobachtungskammem und/oder jeweils entsprechende Zu-/Abläufe oder Durchgangslöcher der einzelnen Mikrofluid-Vorrichtungen vorzugsweise im Raster einer 96-, 384- oder 1536-Well Platte, d.h. mit Abständen von 9mm, 4,5mm oder 2,25mm angeordnet sein.

So kann z.B. die Anordnung eine Bodenplatte und eine Deckplatte umfassen, welche flüssigkeitsdicht miteinander verbunden sind, wobei die Deckplatte Aussparungen aufweist, die mindestens zwei Beobachtungskammem und mindestens vier Reservoire bilden, wobei die Beobachtungskammem jeweils mindestens zwei Reservoire verbinden, die Mikrofluid-Vorrichtung befüllbar ist und die Querschnittsfläche der Beobachtungskammer an der Mündung der Beobachtungskammer in eines der Reservoire mindestens 5mal, insbesondere mindestens 20mal, kleiner ist als die maximale Querschnittsfläche des Reservoirs parallel zu dieser Querschnittsfläche der Beobachtungskammer.

Weiterhin betrifft die Erfindung ein Verfahren zur Erzeugung von diffusiv aufgebauten Gradienten, welches folgende Schritte umfasst:
Bereitstellen einer Mikrofluid-Vorrichtung mit zwei Reservoiren und einer Beobachtungskammer, die die Reservoire verbindet, insbesondere einer Mikrofluid-Vorrichtung wie oben beschrieben,
Befüllen der Reservoire und der Beobachtungskammer mit einer neutralen Flüssigkeit;

Hinzufügen einer chemischen Lösung bekannter Konzentration in ein Reservoir der Mikrofluid-Vorrichtung;
flüssigkeitsdichtes Verschließen des Reservoirs, so dass bei einer Druckbeaufschlagung der Flüssigkeit in dem Reservoir mit dem 10-fachen ihrer Gewichtskraft die absolute Kompression in dem Reservoir maximal 10% des Volumens der Beobachtungskammer beträgt;
wobei die Mikrofluid-Vorrichtung so ausgebildet ist, dass ein über mindestens zwei Tage stabiler diffusiver Gradient aufgebaut wird.

Unter einer neutralen Flüssigkeit versteht man grundsätzlich jede Flüssigkeit, die keine chemische Reaktion mit einer anderen Flüssigkeit (eine chemische Lösung, das Chemokin) und/oder den biologischen Bestandteilen der Probe verursacht. Das Chemokin kann insbesondere in der Flüssigkeit gelöst sein, welche in eines der beiden Reservoire der Mikrofluid-Vorrichtung gefüllt wird und kann dadurch in die Beobachtungskammer diffundieren. Die neutrale Flüssigkeit kann z.B. Reinstwasser oder speziell gemischtes Zellnährmedium sein, dem etwa bestimmte Bestandteile fehlen, wie z.B. FCS.

Die Flüssigkeit, die sich nach dem Hinzufügen einer chemischen Lösung in einem Reservoir befindet, umfasst die neutrale Flüssigkeit in dem Reservoir und die chemische Lösung in diesem Reservoir. Die Gewichtskraft dieser Flüssigkeit ist ihre Gewichtskraft an der Erdoberfläche.

Ist ein Gradient über mindestens zwei Tage stabil, verändert sich sein Gefälle innerhalb von 48 Stunden um nicht mehr als 8%, insbesondere um nicht mehr als 5%.

### Zusätzlich kann obiges Verfahren noch folgenden Schritt umfassen:

Befüllen der Beobachtungskammer mit Zellen.

Weiterhin ist es auch möglich, dass das Befüllen der Beobachtungskammer mit Zellen vor dem Befüllen mit der neutralen Flüssigkeit stattfindet. Es ist zusätzlich auch möglich, dass ein weiteres Befüllen der Beobachtungskammer mit Zellen durchgeführt wird.

Insbesondere kann innerhalb des obigen Verfahrens die Mikrofluid-Vorrichtung mit zwei Reservoiren und einer Beobachtungskammer bereitgestellt werden, wobei das Volumen jedes Reservoirs mindestens 100mal, insbesondere mindestens 200mal, größer als das Volumen der Beobachtungskammer ist und/oder die Querschnittsfläche der Beobachtungskammer an der Mündung der Beobachtungskammer in eines der Reservoire mindestens 5mal , insbesondere mindestens 20mal, kleiner als die maximale Querschnittsfläche des Reservoirs parallel zu dieser Querschnittsfläche der Beobachtungskammer ist und/oder die Länge der Beobachtungskammer zwischen 100µm und 5mm, insbesondere zwischen 500µm und 3mm, beträgt.

Femer kann eine der oben beschriebenen Mikrofluid-Vorrichtungen zur Untersuchung von Zellmigrationen, insbesondere bei diffusiv aufgebauten Gradienten, verwendet werden.

Die Erfindung umfasst außerdem eine Mikrofluid-Vorrichtung, insbesondere eine der oben beschriebenen Mikrofluid-Vorrichtungen, mit einer Bodenplatte und einer Deckplatte, wobei die Deckplatte eine Aussparung aufweist und mit der Bodenplatte flüssigkeitsdicht verbunden ist, so dass die Aussparung eine Beobachtungskammer bildet, wobei die Beobachtungskammer teilweise mit Gel gefüllt ist. Vorzugsweise kann das Gel ein Matrigel, Kollagen-Gel oder Agarose (z.B. SeaPrep® Agarose) sein. Insbesondere kann die Beobachtungskammer eine Querschnittsfläche zwischen 0,05mm² und 0,4mm², vorzugsweise zwischen 0,075mm² und 0,1mm², und eine Länge zwischen 0,5mm und 5mm, vorzugsweise zwischen 1 mm und 2,5mm, aufweisen.

Weiterhin betrifft die Erfindung ein Verfahren zur Herstellung obiger Mikrofluid-Vorrichtung, welches folgende Schritte umfasst:
Bereitstellen einer Bodenplatte und einer Deckplatte, wobei die Deckplatte Aussparungen aufweist;
teilweises Auffüllen der Aussparungen mit einem Gel;
flüssigkeitsdichtes Verbinden der Boden- und Deckplatte.

### Vorzugsweise kann das Verfahren noch folgenden Schritt umfassen:

Befüllen einer Aussparung, insbesondere durch einen Zu-/Ablauf, mit Zellen.

Das Auffüllen der Aussparungen mit einem Gel kann beispielsweise dadurch erreicht werden, dass man die Aussparungen mit Gel bepinselt oder bedruckt, z.B. mit Micro-Contact-Printing, wobei der Stempel aus PDMS (Polydimethylsiloxan), einem 2-Komponenten-Silikon, bestehen kann.

In einer vorteilhaften Weiterbildung kann die Deckplatte mehrere Aussparungen aufweisen, so dass mindestens zwei Reservoire und eine Beobachtungskammer, die die Reservoire verbindet, gebildet werden. Insbesondere kann der Zu-/Ablauf durch die Deckplatte in ein Reservoir führen und die Mikrofluid-Vorrichtung befüllbar sein. Weiterhin kann die Querschnittsfläche der Beobachtungskammer an der Mündung der Beobachtungskammer in eines der Reservoire mindestens 5mal kleiner, insbesondere mindestens 20mal kleiner, sein als die maximale Querschnittsfläche des Reservoirs parallel zu dieser Querschnittsfläche der Beobachtungskammer. Weiterhin kann diese Weiterbildung ein oder mehrere Merkmale der oben beschriebenen Mikrofluid-Vorrichtungen umfassen.

Ein Vorteil dieser Mikrofluid-Vorrichtung und des zugehörigen Verfahrens besteht darin, dass das Gel in der Beobachtungskammer für Zellen eine natürliche Umgebung simulieren kann und daher ihre Migration unter vergleichsweise realen Bedingungen ablaufen kann. Einen weiteren Vorteil stellt die Tatsache dar, dass die Zellen nicht gleichzeitig mit dem Gel in die Beobachtungskammer gefüllt werden müssen, sondern die Zellen in wässrigem Medium eingefüllt werden können, was die Durchführbarkeit der Experimente insbesondere bei kleinen Flüssigkeitsvolumina vereinfacht.

Weitere Vorteile und Merkmale werden nachfolgend an Hand der Figuren beispielhaft erläutert.
- Figur 1: zeigt ein Beispiel für eine Anordnung aus drei Mikrofluid-Vorrichtungen zur Erzeugung diffusiv aufgebauter Gradienten mit jeweils zwei Reservoiren, einer Beobachtungskammer und einem Kanal
- Figur 2: ist eine Draufsicht der Anordnung nach Figur 1 mit einem zusätzlichen Kanal pro Mikrofluid-Vorrichtung
- Figur 3: zeigt eine Draufsicht als Beispiel für eine nicht erfindungsgemäße Anordnung aus drei Mikrofluid-Vorrichtungen zur Erzeugung diffusiv aufgebauter Gradienten mit jeweils vier Reservoiren und einer Beobachtungskammer
- Figur 4: zeigt eine Ausführungsform für Zu-/Abläufe
- Figur 5: ist eine Seitenansicht einer Ausführungsform mit konischen Zu/Abläufen
- Figur 6: ist ein Ausschnitt einer Seitenansicht einer Mikrofluid-Vorrichtung mit zwei Reservoiren und einer Beobachtungskammer
- Figur 7a: ist eine Draufsicht auf eine Beobachtungskammer mit zweispaltigen Zelladhäsionsflächen
- Figur 7b: ist eine Draufsicht auf eine Beobachtungskammer mit einspaltigen Zelladhäsionsflächen
- Figur 7c: ist eine Draufsicht auf eine Beobachtungskammer mit rautenförmigen Zelladhäsionsflächen
- Figur 7d: ist eine Draufsicht auf eine Beobachtungskammer dreieckigen Zelladhäsionsflächen
- Figur 8: ist eine Draufsicht auf eine Anordnung aus 96 Mikrofluid-Vorrichtungen zur Erzeugung diffusiv aufgebauter Gradienten mit jeweils zwei Reservoiren, einer Beobachtungskammer und einem Kanal
- Figur 9a: ist eine Draufsicht als Beispiel für eine Anordnung aus drei Mikrofluid-Vorrichtungen zur Erzeugung diffusiv aufgebauter Gradienten mit jeweils zwei Reservoiren, einer Beobachtungskammer, einem Kanal und einem zusätzlichen Kanal
- Figur 9b: ist eine perspektivische Ansicht der Anordnung aus Figur 9a
- Figur 10a: zeigt eine Draufsicht als Beispiel für eine Mikrofluid-Vorrichtung zur Erzeugung diffusiv aufgebauter Gradienten mit einem Reservoir, einer Beobachtungskammer und einem weiteren Reservoir mit drei Kanälen
- Figur 10b: ist eine perspektivische Ansicht der Mikrofluid-Vorrichtung aus Figur 10a
- Figur 11: zeigt in einer perspektivischen Ansicht eine Beobachtungskammer, die teilweise mit einem Gel und mit Zellen gefüllt ist

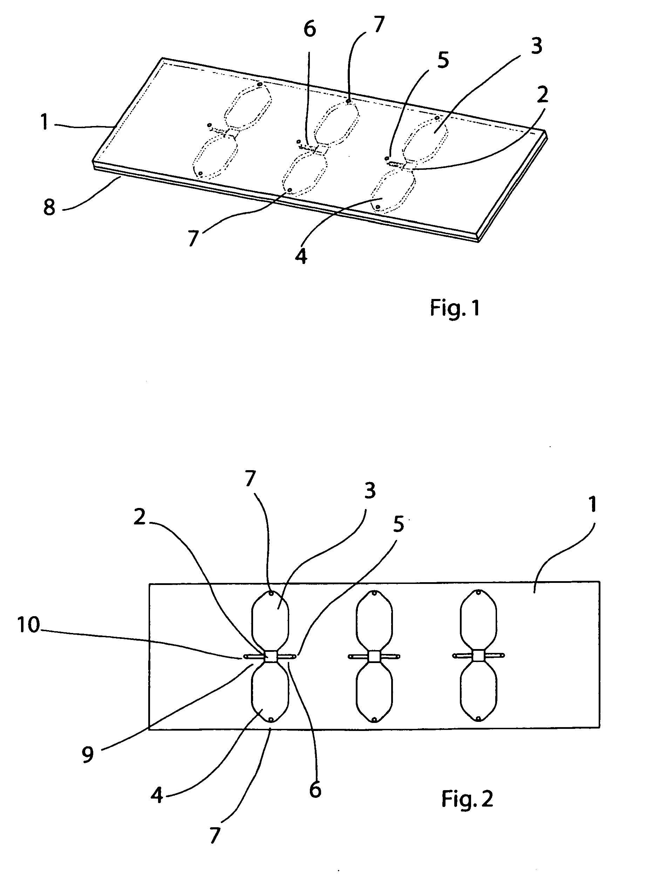

Die erfindungsgemäße Mikrofluid-Vorrichtung und das entsprechende Verfahren zur Erzeugung diffusiv aufgebauter Gradienten werden nachfolgend an Hand der in den Figuren 1 bis 11 gezeigten Beispiele erläutert.

Die Anordnung in Figur 1 hat etwa die Außenmaße eines Objekträgers und umfasst eine Deckplatte 1 und eine Bodenplatte 8 mit drei Mikrofluid-Vorrichtungen mit jeweils zwei Reservoiren 3 und 4, einer Beobachtungskammer 2 und einem Kanal 6. Dabei dient das erste Reservoir 3 als Senke und das zweite Reservoir 4 als Quelle für das Chemokin. Die Beobachtungskammer 2 verbindet die Reservoire 3 und 4 und besitzt eine wesentlich geringere Höhe als die Reservoire 3 und 4. In der Beobachtungskammer werden die Reaktionen z.B. von Zellen auf den durch Diffusion zwischen Senke und Quelle entstehenden Gradienten beobachtet. Als erstes werden die Zellen in den Zu-/Ablauf 5 durch den Kanal 6 in die Beobachtungskammer gefüllt. Dies geschieht so, dass nur die Beobachtungskammer mit Zellsuspension gefüllt ist. Danach wird der Zu-/Ablauf 5 flüssigkeits- und luftdicht verschlossen. Nach einer Wartezeit für die Zelladhäsion werden Quelle 4 und Senke 3 mit einer neutralen Flüssigkeit durch die Zu-/Abläufe 7 befüllt. Danach wird Chemokin in die Quelle 4 durch den Zu-/Ablauf 7 hinzugegeben, wobei die überschüssige Flüssigkeit durch den Zu-/Ablauf 7 in der Senke 3 entfernt wird. Danach werden die beiden Zu-/Abläufe 7 flüssigkeits- und luftdicht verschlossen. Auf diese Weise ist die Anordnung für die Chemotaxis-Untersuchung vorbereitet.

Auf Grund der Zellwanderungsgeschwindigkeit von 20µm/h und eines mikroskopischen Beobachtungsbereichs von etwa 1 mm², ist die bevorzugte Beobachtungsfläche, das ist der Boden der Beobachtungskammer, in diesem Beispiel bei einer 5-fachen Vergrößerung mit einem 5xObjektiv, typischerweise 0,5mm x 0,5mm bis 3mm x 3mm groß. Neben einer quadratischen Form der Beobachtungsfläche kann auch eine beliebige rechteckige Form vorteilhaft sein.

Figur 2 zeigt die gleiche Anordnung wie in Figur 1, nur dass hier jede Mikrofluid-Vorrichtung einen weiteren Kanal 9 mit jeweils einem Zu-/Ablauf 10 aufweist. Dadurch lässt sich die Beobachtungskammer 2 einfacher mit Zellen befüllen und diese Zellen sich nach einer bestimmten Adhäsionswartezeit einfacher hinausspülen.

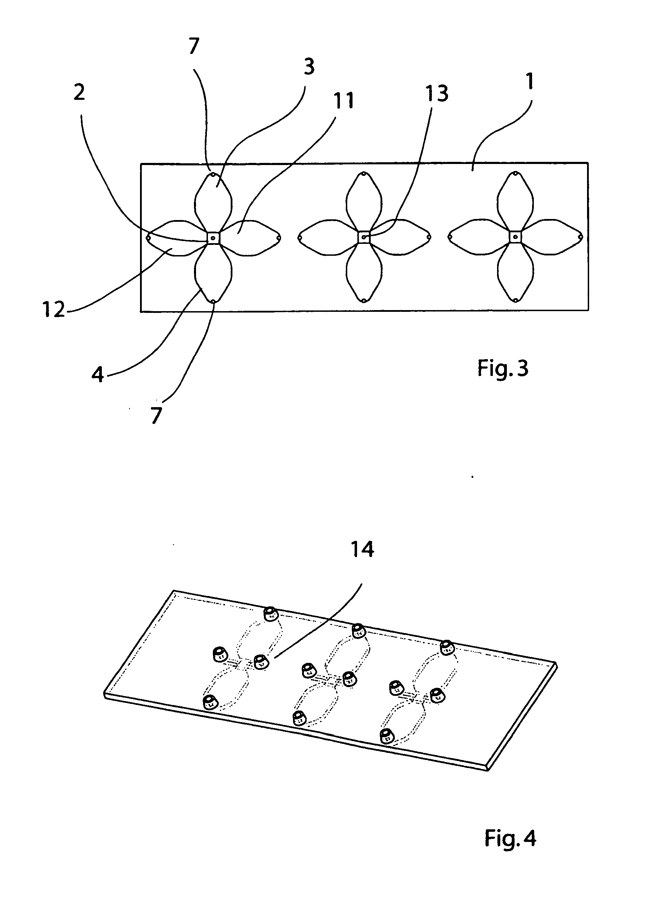

Figur 3 zeigt ein Beispiel für eine nicht erfindungsgemäße Anordnung zur Erzeugung zweier senkrecht zueinander stehenden, diffusiv aufgebauten Gradienten. Die Anordnung weist hier drei Mikrofluid-Vorrichtungen mit jeweils zwei Senken 3 und 11 und zwei Quellen 4 und 12 auf, wobei sich jeweils eine Quelle und eine Senke gegenüberliegen. Weiterhin liegt zwischen den Reservoiren 3, 4, 11 und 12, die jeweils einen Zu-/Ablauf 7 besitzen, die Beobachtungskammer 2. Zur Befüllung der Beobachtungskammer ist in deren Decke zentral ein Durchgangsloch 13 eingebracht.

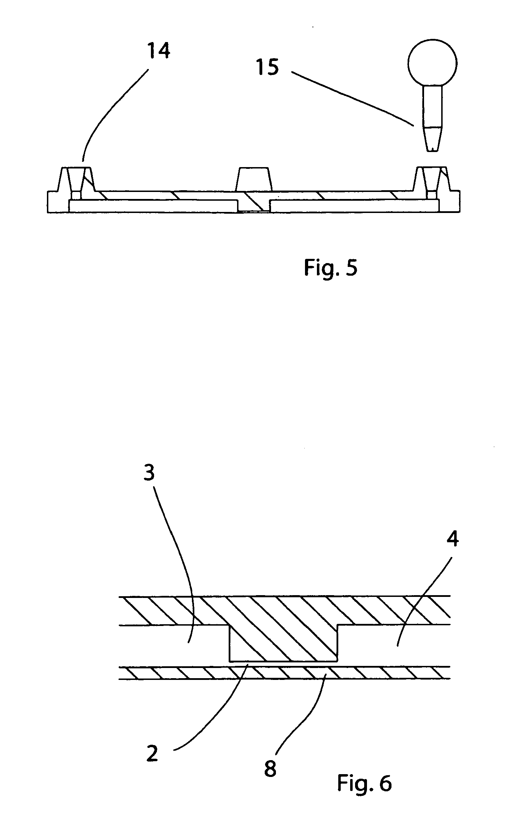

Figuren 4 und 5 zeigen eine spezielle Ausführungsform 14 für Zu-/Abläufe 7. Hier laufen die Zu-/Abläufe nach unten konisch zu, so dass diese mit einem entsprechenden, ebenfalls konisch geformten Stecker 15 flüssigkeits- und luftdicht abgeschlossen werden können.

Figur 6 ist eine vergrößerte Darstellung eines Querschnitts einer Mikrofluid-Vorrichtung mit zwei Reservoiren 3 und 4 und einer Beobachtungskammer 2 zwischen den Reservoiren 3 und 4. Beide Reservoire 3 und 4 sind deutlich höher als der Beobachtungsbereich 2, der Querschnitt der Beobachtungskammer 2 ist also viel kleiner als die Querschnitte der Reservoire 3 und 4, was zu einem langsamen diffusiven Konzentrationsausgleich zwischen den Reservoiren führt.

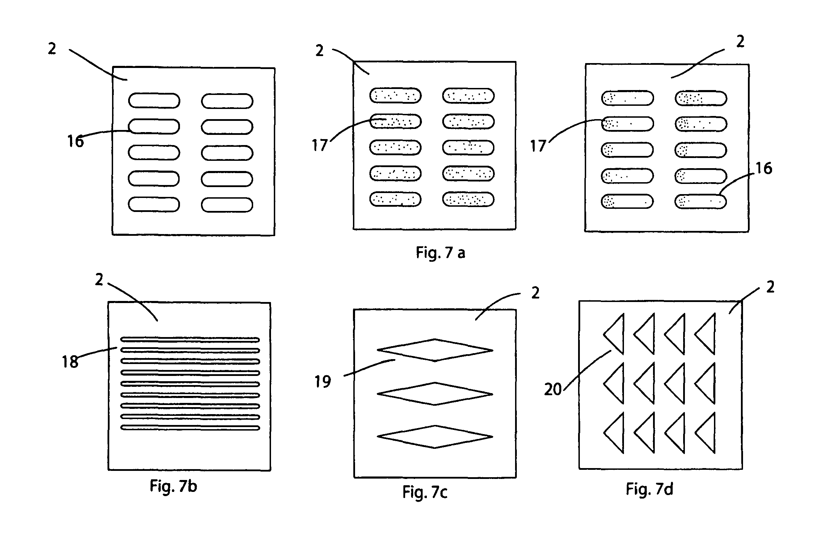

Figur 7a stellt eine Draufsicht auf den Beobachtungsbereich 2 dar. Links grenzt die Quelle 4, rechts die Senke 3 an (nicht dargestellt). Auf dem Beobachtungsbereich befinden sich längliche Flächen 16 für Zelladhäsion, die durch eine Oberflächenstrukturierung und/oder -behandlung präpariert wurden (Figur links). Der Rest der Oberfläche ist nicht für eine Zelladhäsion geeignet. Die Zellen 17 werden in die Beobachtungskammer gespült (z.B. über einen Kanal 6). Nach einer Wartezeit für die Adhäsion werden nicht adhärierte Zellen aus dem Bereich gespült (z.B. über einen Kanal 10), so dass nur die Flächen 16 homogen mit Zellen besät sind (Figur in der Mitte). Nun gibt man zur Quelle 4 das Chemokin hinzu. Nach einer Wartezeit zwischen 12 und 48 Stunden wird eine mikroskopische Aufnahme durchgeführt, um zu kontrollieren, ob die Zelldichte aufgrund der Chemotaxis auf der der Quelle 4 zugewandten Seite der Bereiche 16 zugenommen hat (Figur rechts).

Die Figuren 7b, 7c und 7d zeigen Beobachtungskammem 2 wie in Figur 7a, nur dass hier entweder lange Streifen 18 oder rautenförmige oder dreieckige Flächen 19 und 20 für die Zelladhäsion funktionalisiert, d.h. oberflächenbehandelt, sind. In Figur 7b ist die Breite der Streifen so gewählt, dass die Zellen nur auf einer Linie hintereinander adhärieren können. Mit solchen Strukturen kann man die Zellwanderung z.B. nach Anfärbung mit Kristallviolett o.ä. sogar mit bloßem Auge, also ohne Mikroskop, abschätzen.

Der Vorteil obiger Beobachtungskammem, vorzugsweise sind dies Kanäle, besteht darin, dass sich beim Befüllen die Zellen homogen verteilen, so dass sich ein homogener Zellrasen ausbilden kann. Wichtig ist bei diesen Beispielen, dass die Befüllung schnell (d.h. innerhalb von 30 Sekunden) abläuft und die Beobachtungskammer dabei nicht schräg gehalten wird. Ein langsameres Befüllen könnte dazu führen, dass nur eine Seite der Kammer mit Zellen versehen wird. Die homogene Verteilung der Zellen kann bei chemotaktischen Experimenten wichtig sein, insbesondere wenn die Zellen dazu tendieren, sich gegenseitig zu beeinflussen, was bei einer homogenen Verteilung minimiert werden kann.

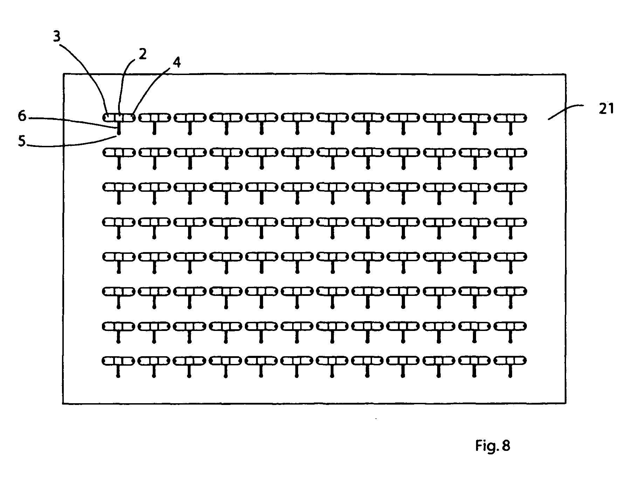

Figur 8 zeigt eine Anordnung mit einer Deckplatte 21 im Format einer 96-Well Platte. Die Zentren der Beobachtungskammem 2 sind in einem quadratischen Gitter mit einem Gitterabstand von 9mm angebracht. Die Anordnung weist acht Reihen mit jeweils zwölf Mikrofluid-Vorrichtungen auf, wobei jede Mikrofluid-Vorrichtung eine Beobachtungskammer 2, zwei Reservoire 3 und 4 und einen Kanal 6 umfasst. In jeder Mikrofluid-Vorrichtung kann - unabhängig von den anderen Mikrofluid-Vorrichtungen - ein Chemotaxis-Experiment durchgeführt werden.

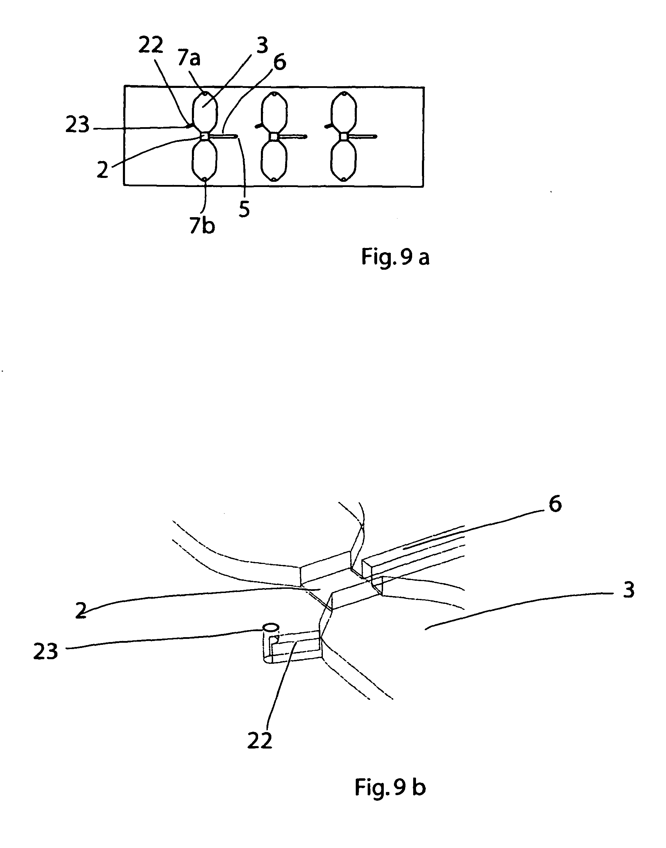

Figur 9 zeigt die Mikrofluid-Vorrichtung aus Figur 1 mit einem zusätzlichen Kanal 22 und einer zusätzlicher Öffnung 23. Dadurch ist es möglich, das Reservoirvolumen durchzuspülen, ohne den diffusiven Gradienten in der Beobachtungskammer 2 zu zerstören. Zu diesem Zweck öffnet man den Zu-/Ablauf 7a des ersten Reservoirs 3 und lässt den Zu-/Ablauf 7b des zweiten Reservoirs und den Zu-/Ablauf 5 des Kanals 6 verschlossen. Durch diese Anordnung entsteht bei einem Spülen über die Öffnung 23 und den Zu-/Ablauf 7a kein Fluss in der Beobachtungskammer 2.

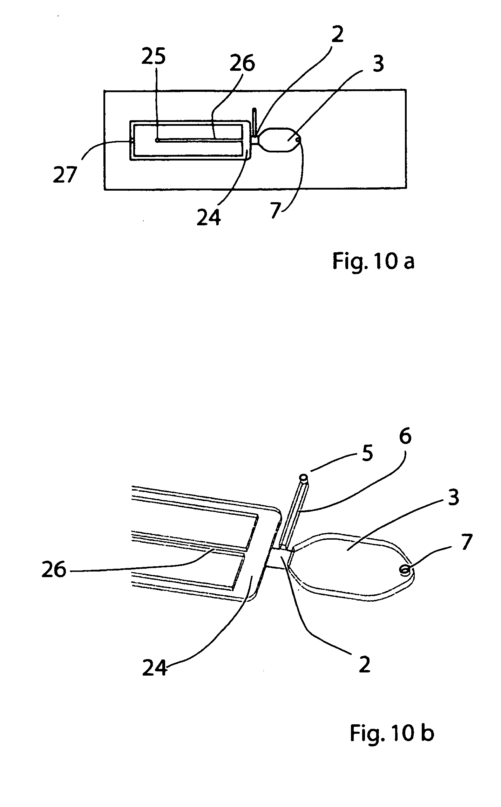

Figur 10 zeigt eine Mikrofluid-Vorrichtung aus Figur 1, wobei ein Reservoir durch eine Kanalstruktur 24, 26 ersetzt ist. Die Kanalstruktur besitzt zwei Zu-/Abläufe 25 und 27. Hält man die Zu-/Abläufe 5 und 7 verschlossen, entsteht bei Fluss durch die Kanalstruktur kein Fluss in der Beobachtungskammer 2. Dadurch ist es möglich, durch stetigen leichten Fluss oder durch immer wieder kurzzeitig gezielten stärkeren Fluss die Konzentration ständig, d.h. über lange Zeiträume, aufrecht zu erhalten.

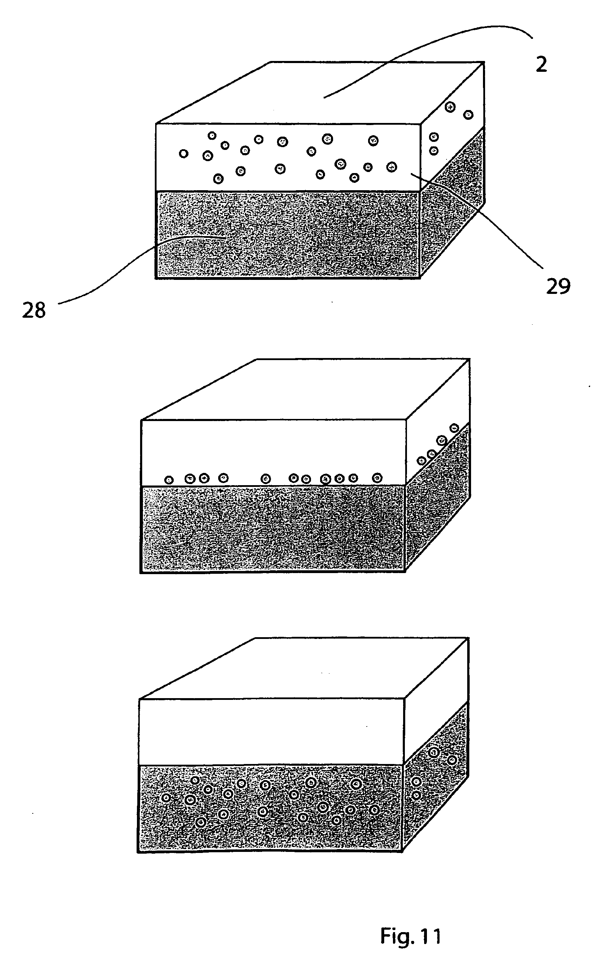

Figur 11 zeigt die Beobachtungskammer 2 in einer speziellen Ausführung. Etwa die Hälfte des Volumens der Beobachtungskammer 2 ist mit Kollagen-Gel 28 aufgefüllt, die obere Hälfte 29 ist im trockenen Zustand leer. Füllt man eine Zellsuspension in die Beobachtungskammer, sinken die Zellen auf das Gel und betten sich aktiv darin ein. Die Zellen finden dort eine naturähnliche Umgebung. Die Migration kann trotz der dreidimensionalen Einbettung in einer Dimension beobachtet werden, da das Gel lediglich den Zeitverlauf nicht aber den räumlichen Verlauf des Gradienten in meßbarem Maße beeinflußt.

Im Folgenden werden anhand von vier bevorzugten Ausführungsbeispielen Verfahrensweisen zur Erzeugung diffusiv aufgebauter Gradienten und deren Nutzung für weitere Analysen erläutert.

### Ausführungssbeispiel 1

Zwei Reservoire sind verbunden durch eine Beobachtungskammer (Kanal) mit einer Grundfläche von 2 x 2mm² und einer Höhe von 0,05mm. Die Höhe der Reservoire beträgt 0,8mm und ihr Grundfläche jeweils ca. 1cm². Somit ist das Volumen jedes Reservoirs mehr als tausendmal so groß wie das Volumen der Beobachtungskammer.

Der Beobachtungsbereich ist mit seiner 2 x 2mm² großen Grundfläche quadratisch, und die Reservoire grenzen jeweils an gegenüberliegende Seiten des Quadrats über die gesamte Seitenlänge der Beobachtungskammer an. Der Boden von Reservoirs und der Beobachtungskammer liegt in derselben Ebene.

Zunächst wird die gesamte Mikrofluid-Vorrichtung, also beide Reservoirs und die Beobachtungskammer mit Reinstwasser gefüllt. Danach füllt man in ein Reservoir (das als Quelle dient) ein Viertel des Reservoirvolumens mit dem Vierfachen der angestrebten Konzentration eines Fluoreszenzfarbstoffes. Die dabei verdrängte Flüssigkeit wird dem zweiten Reservoir (das als Senke dient) entnommen. Danach werden die beiden Zu-/Abläufe der Reservoire luftblasenfrei flüssigkeits- und luftdicht verschlossen. Dadurch erreicht der Farbstoff die Beobachtungskammer nicht durch Strömung, sondern verteilt sich in der Quelle allein durch Diffusion. Nach einiger Zeit erreicht der Farbstoff die Seite der Quelle, die an die Beobachtungskammer grenzt und diffundiert unter Bildung eines Konzentrationsgradienten in die Beobachtungskammer.

Verwendet man z.B. Rhodamin als Farbstoff, so baut sich innerhalb von 4 Stunden ein linearer Gradient zwischen Quell- und Senkreservoir in der Beobachtungskammer auf, den man durch quantitative Fluoreszenzmikroskopie beobachten kann. Die Fluoreszenzmikroskopie erlaubt femer den Nachweis, dass der Gradient über mehr als 48 Stunden quasistabil bleibt, da die Quellkonzentration durch den geringen Stofftransport nur wenig verdünnt wird und die Konzentration im Senkreseryoir dementsprechend nur langsam ansteigt. Dabei entspricht die Konzentration des Farbstoffes in der Beobachtungskammer an den Grenzflächen zu den Reservoirs jeweils etwa der in den Reservoirs vorhandenen Konzentration, und die Konzentration fällt linear in der Beobachtungskammer ab.

### Ausführungsbeispiel 2

Es ist mit einer erfindungsgemäßen Mikrofluid-Vorrichtung mit zwei Reservoiren, einer Beobachtungskammer und einem Kanal auch möglich, eine einfache und sehr reproduzierbare Präparationsprozedur für chemotaktische Migrationsassays durchzuführen.

Zunächst wird dazu die gesamte Mikrofluid-Vorrichtung mit einer neutralen Flüssigkeit befüllt. Danach werden Zellen über einen Zu-/Ablauf durch einen Kanal in die Beobachtungskammer gespült, wo sie an der Oberfläche des Bodens der Beobachtungskammer adhärieren. Der Zu-/Ablauf des Kanals wird dann mit einem Vakuumfett flüssigkeits- und luftdicht verschlossen.

Um eine definierte Konzentration eines Stoffes in einem der Reservoire zu erhalten, ohne dabei die Beobachtungskammer zu fluten, füllt man in ein Reservoir ein Fünftel des Reservoirvolumens einer Flüssigkeit, die die 5-fache Konzentration der eigentlich gewünschten Konzentration hat. Danach werden alle Zu-/Abläufe mit Vakuumfett flüssigkeits- und luftdicht verschlossen.

Durch diffusive Vermischung stellt sich nach ca. 30 Minuten im Reservoir die gewünschte Konzentration ein. Anschließend baut sich in der Beobachtungskammer der Konzentrationsgradient auf.

### Ausführunasbeispiel 3

Die Ausführungen entsprechen denen des Ausführungsbeispiels 2 mit dem Unterschied, dass unmittelbar nach dem Einfüllen des Chemokins in das Quellreservoir das Volumen der Beobachtungskammer durch den bis zu diesem Zeitpunkt noch nicht abgedichteten Zu-/Ablauf des Kanals mit gekühltem flüssigen Matrigel befüllt wird. Das Gel verlangsamt die Diffusion des Chemokins von dem Quellreservoir in die Beobachtungskammer, was zu einem verlangsamten Aufbau des Gradienten, aber zu größerer Zeitstabilität des Gradienten führt. Darüber hinaus wird so die natürliche dreidimensionale Umgebung der Zellen simuliert, wie sie unter realen Bedingungen tatsächlich vorkommt.

### Ausführungsbeispiel 4

Die Ausführungen entsprechen denen des Ausführungsbeispiels 2 mit dem Unterschied, dass die Zellen vor dem Befüllen in den Kanal homogen in Kollagen-Gel aufgelöst werden. Dadurch befinden sich die Zellen in einer 3D-Gelmatrix, die die natürlichen Verhältnisse besser widerspiegeln als 2D-Zellkulturen. Darüber hinaus verlangsamt das Gel die Diffusion, was zu einem verzögerten Aufbau des Gradienten führt, aber eine größere Zeitstabilität des Gradienten begünstigt. Wie schon bei Ausführungsbeispiel 3 wird auf hier die natürliche dreidimensionale Umgebung der Zellen simuliert, wie sie unter realen Bedingungen tatsächlich vorkommt.

### Pipettierprozeduren

Generell ist für das Einfüllen des Chemokins die Tröpfchenmethode bevorzugt. Ausgangspunkt ist hierbei eine vollständig luftblasenfrei befüllte Kanalstruktur.

Voraussetzung für diese Methode ist die gleiche Oberflächenbeschaffenheit und Geometrie der Einfüllöffnungen. Um das Chemokin in ein Reservoir über einen ersten Zu-/Ablauf einzubringen, wird zuerst ein großer Tropfen (ca. 5-faches Volumen der chemokinhaltigen Flüssigkeit) der Flüssigkeit ohne Chemokin auf den zweiten Zu-/Ablauf des Reservoirs gegeben. Anschließend wird das einzubringende Chemokin in kleinen Tröpfchen, langsam auf den ersten Zu-/Ablauf des Reservoirs pipettiert. Die Oberflächenspannung des kleinen Tropfens drückt das Chemokin in die Struktur. Anschließend wird die überstehende Flüssigkeit auf dem zweiten Zu-/Ablauf abgenommen und die Struktur flüssigkeits- und luftdicht abgeschlossen.

Diese hydrostatische Methode kann durch eine physikalische Oberflächenbehandlung (Plasma) unterstützt werden. Wenn die Oberfläche des Zu-/Ablaufs für den großen Tropfen hydrophil (kleiner Grenzwinkel) und die Oberfläche an dem Chemokin-Zu-/Ablauf hydrophob (großer Grenzwinkel) ist, wird das chemokinhaltige Flüssigkeitsvolumen sehr leicht eingesogen. In diesem Fall wird die Pipettierprozedur vereinfacht, da die Volumenunterschiede zwischen dem großem und den kleinen Tropfen viel kleiner sein können.

## Patentansprüche

1. Mikrofluid-Vorrichtung zur Erzeugung diffusiv aufgebauter Gradienten mit einer Bodenplatte (8) und einer Deckplatte (1), wobei
die Deckplatte (1) Aussparungen aufweist und mit der Bodenplatte (8) flüssigkeitsdicht verbunden ist, so dass die Aussparungen mindestens zwei Reservoire (3, 4) und eine Beobachtungskammer (2), die die Reservoire (3, 4) verbindet, bilden,
ein Reservoir (3; 4), insbesondere durch einen Zu-/Ablauf (7) durch die Deckplatte (1), befüllbar ist und
die Querschnittsflächen der Beobachtungskammer (2) an mindestens zwei Mündungen der Beobachtungskammer (2) in jeweils ein Reservoir (3, 4) mindestens 5mal, insbesondere mindestens 20mal, kleiner als die maximale Querschnittsfläche des Reservoirs (3; 4) parallel zu dieser Querschnittsfläche der Beobachtungskammer (2) sind, und
wobei die Mikrofluid-Vorrichtung zusätzlich mindestens einen Kanal (6) mit mindestens einem Zu-/Ablauf (5) umfasst,
wobei der Kanal (6) in die Beobachtungskammer (2) führt, **dadurch gekennzeichnet, daß** der Kanal (6) über eine Seite der Beobachtungskammer (2) in die Beobachtungskammer (2) führt, und wobei die Beobachtungskammer (2) an dieser Seite nicht an ein Reservoir anschließt.

2. Mikrofluid-Vorrichtung nach Anspruch 1, wobei das befüllbare Reservoir (3; 4) einen Zu-/Ablauf (7) aufweist, wobei die minimale Querschnittsfläche des Zu-/Ablaufs (7) zwischen 0,03mm² und 13mm², insbesondere zwischen 0,2mm² und 3,2mm², beträgt.

3. Mikrofluid-Vorrichtung nach Anspruch 1 oder 2, wobei die Querschnittsfläche der Beobachtungskammer (2) zwischen 0,05mm² und 0,4mm², vorzugsweise zwischen 0,075mm² und 0,1mm², liegt und/oder die Querschnittsfläche eines Reservoirs (3; 4) zwischen 0,25mm² und 8mm², vorzugsweise zwischen 0,37mm² und 2mm², liegt.

4. Mikrofluid-Vorrichtung nach einem der vorangegangenen Ansprüche, wobei ein Durchgangsloch (13) durch die Deckplatte (1) und/oder die Bodenplatte (8) in die Beobachtungskammer (2) vorgesehen ist.

5. Mikrofluid-Vorrichtung nach einem der vorangegangenen Ansprüche, wobei der Boden der Beobachtungskammer (2) und der Boden mindestens eines Reservoirs (3; 4) in einer Ebene liegen.

6. Mikrofluid-Vorrichtung nach einem der vorangegangenen Ansprüche, wobei die Bodenplatte (8) mindestens in dem Bereich der Beobachtungskammer (2) aus einem optisch hochwertigen Material besteht.

7. Mikrofluid-Vorrichtung nach einem der vorangegangenen Ansprüche, wobei sich der Boden des Kanals (6) in der gleichen Ebene befindet wie der Boden der Beobachtungskammer (2).

8. Mikrofluid-Vorrichtung nach einem der vorangegangenen Ansprüche, wobei die Höhe und/oder Breite der Beobachtungskammer (2) und/oder der Reservoire (3, 4) konstant ist, insbesondere die Beobachtungskammer (2) und/oder mindestens ein Reservoir (3; 4) die Form eines geraden Prismas mit einer geraden Seitenanzahl besitzt und insbesondere die Beobachtungskammer (2) und/oder mindestens ein Reservoir (3; 4) quaderförmig ausgebildet ist.

9. Mikrofluid-Vorrichtung nach einem der vorangegangenen Ansprüche, welche mindestens zwei weitere Reservoire (11, 12) umfasst, wobei die Reservoire (3, 4, 11, 12) an den Seiten der Beobachtungskammer (2) so angeordnet sind, dass sich jeweils zwei Reservoire (3, 4; 11, 12) gegenüberliegen.

10. Mikrofluid-Vorrichtung nach einem der vorangegangenen Ansprüche, welche wenigstens eine, insbesondere rechteckige (16; 18), rautenförmige (19), dreieckige (20) oder runde, Zelladhäsionsfläche (16; 18; 19; 20) in der Beobachtungskammer (2) aufweist.

11. Mikrofluid-Vorrichtung nach einem der vorangegangenen Ansprüche, welche wenigstens eine Markierung in der Beobachtungskammer (2) zur Bestimmung von Ort und/oder Geschwindigkeit von migrierenden Zellen aufweist.

12. Mikrofluid-Vorrichtung nach einem der vorangegangenen Ansprüche, wobei die Beobachtungskammer (2) teilweise mit Gel gefüllt ist.

13. Mikrofluid-Vorrichtung nach Anspruch 12, wobei das Gel ein Matrigel, Kollagen-Gel, oder Agarose ist.

14. Anordnung zur Erzeugung diffusiv aufgebauter Gradienten mit wenigstens zwei Mikrofluid-Vorrichtungen nach einem der vorangegangenen Ansprüche, wobei die wenigstens zwei Bodenplatten (8) als eine einzige Bodenplatte (8) ausgebildet sind und die wenigstens zwei Deckplatten (1) als eine einzige Deckplatte (1) ausgebildet sind.

15. Verfahren zur Erzeugung von diffusiv aufgebauten Gradienten, welches folgende Schritte umfasst:
Bereitstellen einer Mikrofluid-Vorrichtung nach einem der Ansprüche 1 bis 13,
Befüllen der Reservoire (3, 4) und der Beobachtungskammer (2) mit einer neutralen Flüssigkeit;
Hinzufügen einer chemischen Lösung bekannter Konzentration in ein Reservoir (3; 4) der Mikrofluid-Vorrichtung;
flüssigkeitsdichtes Verschließen des Reservoirs (3; 4), so dass bei einer Druckbeaufschlagung der Flüssigkeit in dem Reservoir (3; 4) mit dem 10-fachen ihrer Gewichtskraft die absolute Kompression in dem Reservoir (3; 4) maximal 10% des Volumens der Beobachtungskammer (2) beträgt;
wobei die Mikrofluid-Vorrichtung so ausgebildet ist, dass ein über mindestens zwei Tage stabiler diffusiver Gradient aufgebaut wird.

16. Verfahren nach Anspruch 15, welches zusätzlich folgenden Schritt umfasst:
Befüllen der Beobachtungskammer (2) mit Zellen, wobei insbesondere das Befüllen mit Zellen nach dem Befüllen mit der neutralen Flüssigkeit und vor dem Hinzufügen der definiert konzentrierten chemischen Lösung stattfindet.

17. Verfahren nach Anspruch 15 oder 16, wobei die Mikrofluid-Vorrichtung mit zwei Reservoiren (3, 4) und einer Beobachtungskammer (2) bereitgestellt wird, wobei das Volumen jedes Reservoirs (3; 4) mindestens 100mal, insbesondere mindestens 200mal, größer als das Volumen der Beobachtungskammer (2) ist und/oder die Querschnittsfläche der Beobachtungskammer (2) an der Mündung der Beobachtungskammer (2) in eines der Reservoire (3; 4) mindestens 5mal, insbesondere mindestens 20mal, kleiner als die maximale Querschnittsfläche des Reservoirs (3; 4) parallel zu dieser Querschnittsfläche der Beobachtungskammer (2) ist und/oder die Länge der Beobachtungskammer (2) zwischen 100µm und 5mm, insbesondere zwischen 500µm und 3mm, beträgt.

## Claims

1. A microfluid device for producing diffusively built gradients comprising a bottom plate (8) and a cover plate (1), wherein
the cover plate (1) has recesses and is connected with the bottom plate (8) in a liquid-tight manner so that the recesses form at least two reservoirs (3, 4) and an observation chamber (2), which connects the reservoirs (3, 4),
a reservoir (3; 4) can be filled, particularly through an inlet/outlet (7) through the cover plate (1), and
the cross-sectional surfaces of the observation chamber (2) at least at two apertures of the observation chamber (2) in each case into one of the reservoirs (3, 4) is at least 5 times, particularly at least 20 times, smaller than the maximum cross-sectional surface of the reservoir (3; 4) in parallel to this cross-sectional surface of the observation chamber (2), and
wherein the microfluid device additionally comprises at least one channel (6) with at least one inlet/outlet (5),
wherein the channel (6) leads into the observation chamber (2), **characterized in that** the channel (6) leads over one side of the observation chamber (2) into the observation chamber (2), and wherein the observation chamber (2) does not adjoin a reservoir at this side.

2. A microfluid device as claimed in claim 1, wherein the reservoir (3; 4) that can be filled comprises an inlet/outlet (7), wherein the minimal cross-sectional surface of the inlet/outlet (7) is between 0.03 mm² and 13 mm², particularly between 0.2 mm² and 3.2 mm².

3. A microfluid device as claimed in claim 1 or 2, wherein the cross-sectional surface of the observation chamber (2) is between 0.05 mm² and 0.4 mm², preferably between 0.075 mm² and 0.1 mm², and/or the cross-sectional surface of a reservoir (3; 4) is between 0.25 mm² and 8 mm², preferably between 0.37 mm² and 2 mm².

4. A microfluid device as claimed in one of the preceding claims, wherein a through hole (13) through the cover plate (1) and/or the bottom plate (8) into the observation chamber (2) is provided.

5. A microfluid device as claimed in one of the preceding claims, wherein the bottom of the observation chamber (2) and the bottom of at least one reservoir (3; 4) are located on one plane.

6. A microfluid device as claimed in at least one of the preceding claims, wherein the bottom plate (8) consists of an optically high-quality material at least in the area of the observation chamber (2).

7. A microfluid device as claimed in one of the preceding claims, wherein the bottom of the channel (6) is located on the same plane as the bottom of the observation chamber (2).

8. A microfluid device as claimed in one of the preceding claims, wherein the height and/or width of the observation chamber (2) and/or the reservoirs (3, 4) is constant, particularly the observation chamber (2) and/or a reservoir (3; 4) has the shape of an even prism with an even number of sides, and particularly the observation chamber (2) and/or at least one reservoir (3; 4) has the shape of a cuboid.

9. A microfluid device as claimed in at least one of the preceding claims, which comprises at least two further reservoirs (11, 12), wherein the reservoirs (3, 4, 11, 12) are arranged on the sides of the observation chamber (2) in a manner that two reservoirs (3, 4; 11, 12) each oppose one another.

10. A microfluid device as claimed in at least one of the preceding claims, which comprises at least one, particularly rectangular (16; 18), rhombical (19), triangular (20) or round, cell adhesion surface (16; 18; 19; 20) in the observation chamber (2).

11. A microfluid device as claimed in one of the preceding claims, which comprises at least one marking in the observation chamber (2) to determine the location and/or velocity of migrating cells.

12. A microfluid device as claimed in one of the preceding claims, wherein the observation chamber (2) is partially filled with gel.

13. A microfluid device as claimed in claim 13, wherein the gel is a matrigel, collagen gel or agarose.

14. An arrangement for producing diffusively built gradients with at least two microfluid devices as claimed in one of the preceding claims, wherein the at least two bottom plates (8) are formed as one single bottom plate (8) and the at least two cover plates (1) are formed as one single cover plate (1).

15. A method of producing diffusively built gradient, comprising the following steps:
providing a microfluid device according to one of claims 1 to 13,
filling the reservoirs (3, 4) and the observation chamber (2) with a neutral liquid;
adding a chemical solution of a known concentration into a reservoir (3; 4) of the microfluid device;
liquid-tight locking of the reservoir (3; 4) so that in the case of a pressure load of the liquid in the reservoir (3; 4) with 10 times its weight, the absolute compression in the reservoir is a maximum of 10 % of the volume of the observation chamber (2);
wherein the microfluid device is formed such that a diffusive gradient that is stable over at least two days is built up.

16. A method as claimed in claim 15, which additionally comprises the following step:
filling the observation chamber (2) with cells, wherein particularly the filling with cells takes place after the filling with the neutral liquid and before adding the defined concentrated chemical solution.

17. A method as claimed in claim 15 or 16, wherein the microfluid device is provided with two reservoirs (3, 4) and one observation chamber (2), wherein the volume of each reservoir (3; 4) is at least 100 times, particularly at least 200 times larger than the volume of the observation chamber (2) and/or the cross-sectional surface of the observation chamber (2) at the aperture of the observation chamber (2) into one of the reservoirs (3; 4) is at least 5 times, particularly at least 20 times smaller than the maximum cross-sectional surface of the reservoir (3; 4) in parallel to this cross-sectional surface of the observation chamber (2) and/or the length of the observation chamber (2) is between 100 µm and 5 mm, particularly between 500 µm and 3 mm.

## Revendications

1. Appareil microfluidique pour produire des gradients diffusifs, avec une plaque de base (8) et une plaque de recouvrement (1), étant précisé
que la plaque de recouvrement (1) présente des évidements et est reliée à la plaque de base (8) de manière étanche au liquide, de sorte que les évidements forment au moins deux réservoirs (3 ;4) et une chambre d'observation (2) qui relie les réservoirs (3 ; 4),
qu'un réservoir (3 ; 4) est apte à être rempli, en particulier grâce à une arrivée/sortie (7) prévue à travers la plaque de recouvrement (1), et
que les surfaces de section de la chambre d'observation (2) au niveau d'au moins deux points où la chambre d'observation (2) débouche dans un réservoir respectif (3 ; 4) sont au moins 5 fois, en particulier au moins 20 fois plus petites que la surface de section maximale du réservoir (3 ; 4) parallèlement à cette surface de section de la chambre d'observation (2), et
étant précisé que l'appareil microfluidique comprend en supplément au moins un conduit (6) avec au moins une arrivée/sortie (5),
étant précisé que le conduit (6) mène dans la chambre d'observation (2), **caractérisé en ce que** le conduit (6) mène dans la chambre d'observation (2) par un côté de celle-ci, et que ladite chambre d'observation (2), sur ce côté, ne fait pas suite à un réservoir.

2. Appareil microfluidique selon la revendication 1, étant précisé que le réservoir (3 ; 4) apte à être rempli présente une arrivée/sortie (7), et que la surface de section minimale de l'arrivée/sortie (7) est située entre 0,03 mm² et 13 mm², en particulier entre 0,2 mm² et 3,2 mm².

3. Appareil microfluidique selon la revendication 1 ou 2, étant précisé que la surface de section de la chambre d'observation (2) est située entre 0,05 mm² et 0,4 mm², de préférence entre 0,075 mm² et 0,1 mm² et/ou la surface de section d'un réservoir (3 ; 4) est située entre 0,25 mm² et 8 mm², de préférence entre 0,37 mm² et 2 mm².

4. Appareil microfluidique selon l'une des revendications précédentes, étant précisé qu'un trou traversant (13) est prévu dans la plaque de recouvrement (1) et/ou dans la plaque de base (8) jusqu'à la chambre d'observation (2).

5. Appareil microfluidique selon l'une des revendications précédentes, étant précisé que le fond de la chambre d'observation (2) et le fond d'au moins un réservoir (3 ; 4) sont situés dans un même plan.

6. Appareil microfluidique selon l'une des revendications précédentes, étant précisé que la plaque de base (8) se compose, au moins dans la zone de la chambre d'observation (2), d'un matériau à valeur optique élevée.

7. Appareil microfluidique selon l'une des revendications précédentes, étant précisé que le fond du conduit (6) se trouve dans le même plan que le fond de la chambre d'observation (2).

8. Appareil microfluidique selon l'une des revendications précédentes, étant précisé que la hauteur et/ou la largeur de la chambre d'observation (2) et/ou des réservoirs (3 ; 4) sont constantes, en particulier la chambre d'observation (2) et/ou au moins un réservoir (3 ; 4) ont la forme d'un prisme droit avec un nombre de côtés pair et en particulier la chambre d'observation (2) et/ou au moins un réservoir (3 ; 4) sont parallélépipédiques.

9. Appareil microfluidique selon l'une des revendications précédentes, qui comprend au moins deux réservoirs supplémentaires (11, 12), étant précisé que les réservoirs (3, 4, 11, 12) sont disposés sur les côtés de la chambre d'observation (2) de telle sorte que les réservoirs (3, 4 ; 11, 12) se font face respectivement deux à deux.

10. Appareil microfluidique selon l'une des revendications précédentes, qui présente dans la chambre d'observation (2) au moins une surface d'adhésion cellulaire (16 ; 18 ; 19 ; 20) en particulier rectangulaire (16 ; 18), en forme de losange (19), triangulaire (20) ou ronde.

11. Appareil microfluidique selon l'une des revendications précédentes, qui présente au moins un marquage dans la chambre d'observation (2) pour définir la position et/ou la vitesse de cellules migrantes.

12. Appareil microfluidique selon l'une des revendications précédentes, étant précisé que la chambre d'observation (2) est en partie remplie de gel.

13. Appareil microfluidique selon la revendication 12, étant précisé que le gel est constitué par du Matrigel, du gel de collagène ou de l'agarose.

14. Dispositif pour produire des gradients diffusifs, avec au moins deux appareils microfluidiques selon l'une des revendications précédentes, étant précisé que les deux plaques de base (8), ou plus, sont conçues sous la forme d'une plaque de base unique (8) tandis que les deux plaques de recouvrement (1), ou plus, sont conçues comme une plaque de recouvrement unique (1).

15. Procédé pour produire des gradients diffusifs, qui comprend les étapes qui consistent :
à prévoir un appareil microfluidique selon l'une des revendications 1 à 13,
à remplir les réservoirs (3 ; 4) et la chambre d'observation (2) avec un liquide neutre ;
à ajouter une solution chimique à concentration connue dans un réservoir (3 ; 4) de l'appareil microfluidique ;
à fermer le réservoir (3 ; 4) de manière étanche au liquide de telle sorte qu'en présence d'une contrainte de pression du liquide dans le réservoir (3 ; 4) avec 10 fois son poids, la compression absolue dans le réservoir (3 ; 4) représente au maximum 10% du volume de la chambre d'observation (2), étant précisé que l'appareil microfluidique est conçu pour que soit formé un gradient diffusif stable sur au moins deux jours.

16. Procédé selon la revendication 15, qui comprend en supplément les étapes suivantes :
remplissage de la chambre d'observation (2) avec des cellules, étant précisé en particulier que le remplissage avec des cellules a lieu après le remplissage avec le liquide neutre et avant l'adjonction de la solution chimique à concentration définie.

17. Procédé selon la revendication 15 ou 16, étant précisé que l'appareil microfluidique est prévu avec deux réservoirs (3 ; 4) et une chambre d'observation (2), que le volume de chaque réservoir (3 ; 4) est au moins 100 fois, en particulier au moins 200 fois supérieur au volume de la chambre d'observation (2) et/ou que la surface de section de la chambre d'observation (2) à l'endroit où celle-ci débouche dans l'un des réservoirs (3 ; 4) est au moins 5 fois, en particulier au moins 20 fois plus petite que la surface de section maximale du réservoir (3 ; 4) parallèlement à cette surface de section de la chambre d'observation (2) et/ou que la longueur de la chambre d'observation (2) est située entre 100 µm et 5 mm, en particulier entre 500 µm et 3 mm.
